# EUROPEAN PATENT APPLICATION

(11) **EP 3 566 648 A1**
(43) Date of publication of application: **13.11.2019**
(21) Application number: 17889806.0
(22) Date of filing: 04.01.2017
(51) Int. Cl.: A61B 5/11, A61B 5/107

(54) **INFORMATION PROCESSING DEVICE, INFORMATION PROCESSING SYSTEM, AND INFORMATION PROCESSING METHOD**

(71) Applicant: Fujitsu Limited, Kawasaki-shi, Kanagawa 211-8588 (JP)
(72) Inventor: OYA, Takuro, Kawasaki-shi Kanagawa 211-8588 (JP); YAGINUMA, Yoshinori, Kawasaki-shi Kanagawa 211-8588 (JP)
(74) Representative: Hoffmann Eitle
(86) International application number: PCT/JP2017/000046
(87) International publication number: WO 2018/127947

(57) **Abstract**

There is provided an information processing device having an extraction unit that extracts data of a characteristic part concerning a variation of a movement of a subject from data that is obtained from a sensor attached to the subject and indicates the movement of the subject, an action estimation unit that estimates an action of the subject with reference to a storage unit that stores an action pattern of the subject, the action corresponding to the extracted data of the characteristic part, and a form estimation unit that estimates a form of an action of the subject, the form corresponding to the extracted data of the characteristic part.

## Description

### TECHNICAL FIELD

The present invention relates to an information processing device, an information processing system, and an information processing method.

### BACKGROUND ART

There are various approaches for quantifying an action of a human or an animal. For example, technologies for detecting a posture of a human body by attaching a plurality of sensors to a plurality of portions of the human body are known (for example, refer to Patent Document 1 or 2).

However, values detected by the sensors contain noise data that is different from the actual action. Therefore, technologies for removing the noise data from data obtained from the sensors have been proposed. For example, a technology for counting the number of steps by detecting walking and ignoring walking data with an amplitude equal to or less than a predetermined threshold value is known (for example, refer to Patent Document 3).

### CITATION LIST

### PATENT DOCUMENT

Patent Document 1: Japanese Laid-open Patent Publication No. 2011-78728
Patent Document 2: Japanese Laid-open Patent Publication No. 2006-326174
Patent Document 3: Japanese Laid-open Patent Publication No. 2012-65749

### SUMMARY OF INVENTION

### TECHNICAL PROBLEM

However, the determination using the threshold value may cause a case where it is difficult to determine whether or not data obtained from the sensors is the noise data. For example, a person in a poor physical condition or an aged person may not make a very clear motion. In that case, determining with reference to a predetermined threshold value whether or not the data obtained from the sensors is the noise may cause an error in the determination result and the action may not be able to be quantified correctly.

Therefore, an object of one aspect of the present invention is to quantify the action correctly.

### SOLUTION TO PROBLEM

In one aspect of the embodiment, there is provided an information processing device having an extraction unit that extracts data of a characteristic part concerning a variation of a movement of a subject from data that is obtained from a sensor attached to the subject and indicates the movement of the subject, an action estimation unit that estimates an action of the subject with reference to a storage unit that stores action patterns of the subject, the action corresponding to the extracted data of the characteristic part, and a form estimation unit that estimates a form of the estimated action of the subject, the form corresponding to the extracted data of the characteristic part.

### ADVANTAGEOUS EFFECTS OF INVENTION

One aspect of the present invention is to be able to quantify the action correctly.

### BRIEF DESCRIPTION OF DRAWINGS

FIG. 1 is a diagram for illustrating an example of a problem of form estimation using a sensor.
FIG. 2 is a diagram for illustrating the form estimation using the sensor according to an embodiment.
FIG. 3 is a diagram that illustrates an example of a hardware configuration of an information processing device according to the embodiment.
FIG. 4 is a diagram that illustrates an example of an information processing system according to the embodiment.
FIG. 5 is a diagram that illustrates an example of a functional configuration of the information processing device according to the embodiment.
FIG. 6 is a diagram that illustrates an example of a sensor management table according to the embodiment.
FIG. 7 is a diagram that illustrates an example of a body type model table according to the embodiment.
FIG. 8 is a diagram that illustrates an example of a data specifying table according to the embodiment.
FIG. 9 is a diagram that illustrates an example of a position specifying table according to the embodiment.
FIG. 10 is a diagram that illustrates an example of a combination table according to the embodiment.
FIG. 11 is a diagram that illustrates an example of an action learning table according the embodiment.
FIG. 12 is a diagram that illustrates an example of a motion range table according to the embodiment.
FIG. 13 is a flowchart that illustrates an example of preprocessing according to the embodiment.
FIG. 14 is a flowchart that illustrates an example of learning processing according to the embodiment.
FIG. 15 is a diagram for illustrating a processing flow in a learning mode according to the embodiment.
FIG. 16 is a diagram that illustrates an example of a data specifying table according to the embodiment.
FIG. 17 is a diagram that illustrates an example of waveform extraction of the data specifying table according to the embodiment.
FIG. 18 is a diagram that illustrates examples of the data specifying table and the action learning table according to the embodiment.
FIG. 19 is a diagram that illustrates an example of a motion range table according to the embodiment.
FIG. 20 is a flowchart that illustrates an example of form estimation processing according to the embodiment.
FIG. 21 is a diagram for illustrating a processing flow in a form estimation mode according to the embodiment.
FIG. 22 is a diagram that illustrates examples of the combination table, the data specifying table, and the position specifying table according to the embodiment.
FIG. 23 is a diagram that illustrates examples of the data specifying table and the action learning table according to the embodiment.
FIG. 24 is a diagram that illustrates examples of the data specifying table and the position specifying table according to the embodiment.
FIG. 25 is a diagram that illustrates an example of the motion range table according to the embodiment.
FIG. 26 is a diagram that illustrates an example of the characteristic part concerning the variation of the movement of the subject according to the embodiment.

### DESCRIPTION OF EMBODIMENTS

Hereinafter, an embodiment of the present invention is described with reference to the attached drawings. Note that, in the description and the drawings, components having substantially the same functional configuration are denoted by the same reference numerals, and redundant description is omitted.

### <Introduction>

Systems that understand a condition and an action of a human by sensing the action and bio-information of the human, and provide a service depending on with the condition and the action, have been proposed. For example, in a rehabilitation scene, it is made possible to make a quantitative determination on the degree of a recovery status and a health condition of a patient by sensing a continuous posture (hereinafter referred to as a "form") of the patient, and estimating and visualizing the form of the patient. In addition, the provision of the service as stated above will be helpful in improving the motivation of the patient itself.

Therefore, it is considered to quantify the action of the human by observing the action using a motion capture for example. In addition, it is also considered to quantify the action of the human by using an image sensor and a wearable sensor.

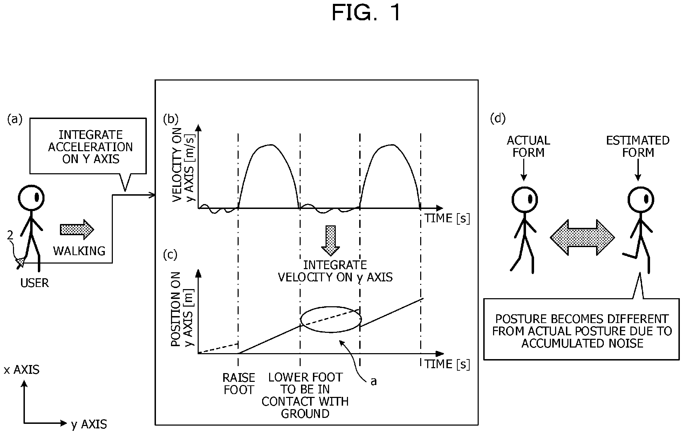

However, in the quantification of the action by using the sensors, there are cases where the form may not be estimated correctly due to effects of noises contained in the sensors. As an example, there is a case to be pointed out where a portion of the human seems moving due to an effect of a drift noise of the wearable sensor although the portion is not moving. As illustrated in FIG. 1(a), the phenomenon is described by taking a case where a user of the system walks with a position specifying sensor 2 (an acceleration sensor) attached on its ankle for example.

By integrating an acceleration in the y axis direction detected by the position specifying sensor 2, a time series variation of a velocity in the y axis direction is calculated as illustrated in FIG. 1(b). An action from raising a foot until lowering the raised foot to be in contact with the ground is a characteristic part having a velocity variation with a shape of an arch (low → high → low).

On the other hand, during a period from raising the foot not attached with the position specifying sensor 2 until lowering the raised foot to be in contact with the ground, the foot attached with the position specifying sensor 2 keeps being in contact with the ground. However, during the period, a waveform is generated as if the foot attached with the position specifying sensor 2 seems moving due to the effect of the drift noise of the wearable sensor although the foot is not moving.

In addition, by integrating the velocity on the y axis, a time series variation of a position in the y axis direction is calculated as illustrated in FIG. 1(c). According to the calculation, the drift noise is accumulated by the integration of the velocity as illustrated in the portion of "a", and a result indicating that a position of the foot is not in contacting with the ground is output although the foot is in contact with the ground. As a result, in contrast to a form of a normal walking illustrated on the left in FIG. 1(d), a form estimated from data of the acceleration detected by the position specifying sensor 2 becomes a posture different from an actual form as illustrated on the right in FIG. 1(d).

Therefore, in an information processing device according to the present embodiment, the noise is removed from data that is obtained from the sensor attached to a subject and indicates the movement of the subject, so as to quantify the action correctly and estimate the form accurately. In the present embodiment, data having the characteristic part is extracted from the data obtained from the sensor in order to suppress the noise as much as possible. For example, in the present embodiment, data of the varying part where an energy of motion transitions as low → high → low (data of the characteristic part) is extracted from the obtained sensor data. Consequently, a range of a short-term energy variation specific to the motion can be specified, and the action can be quantified by using only the sensor data within the specified range.

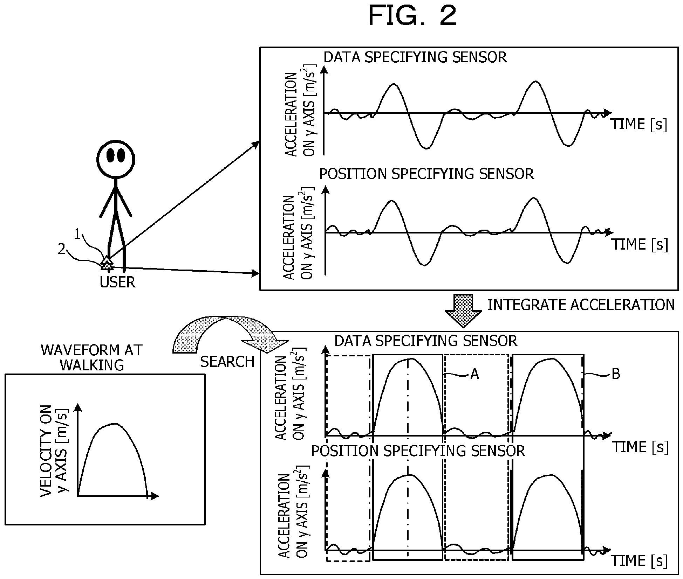

For example, as illustrated in FIG. 2, in a case where the user with a data specifying sensor 1 and a position specifying sensor 2 attached on the right foot walks, accelerations with almost a same value are detected from the both sensors as illustrated in the top and bottom graphs in the upper portion of FIG. 2. In this case, by integrating the obtained accelerations, velocities with almost the same value are calculated as illustrated in the top and bottom graphs in the lower part of FIG. 2. In this case, the information processing device according to the present embodiment extracts velocities in a range A and a range B where the calculated velocity on the y axis transitions as low → high → low. Furthermore, the information processing device quantifies the action of the user on the basis of information of the extracted velocities, and estimates the form of the user. Consequently, the noise is removed from the acceleration detected by each sensor, and the correct form can be estimated. In the case of FIG. 2, in the range A for instance, the information processing device according to the present embodiment estimates the action as "walking" when the velocity of the data specifying sensor 1 and waveform data of a velocity at walking learned beforehand are compared and almost match each other, and estimates a form for the estimated action.

Note that the position specifying sensor 2 may be attached on a portion different from a portion on which the data specifying sensor 1 is attached. In the following description, an acceleration detected by the data specifying sensor 1 is used for extracting velocity information of the characteristic part where the energy of motion transitions as small → large → small within a velocity obtained by integrating the acceleration. On the other hand, an acceleration detected by the position specifying sensor 2 is used for estimating the form of the user.

### [Hardware Configuration of Information Processing Device]

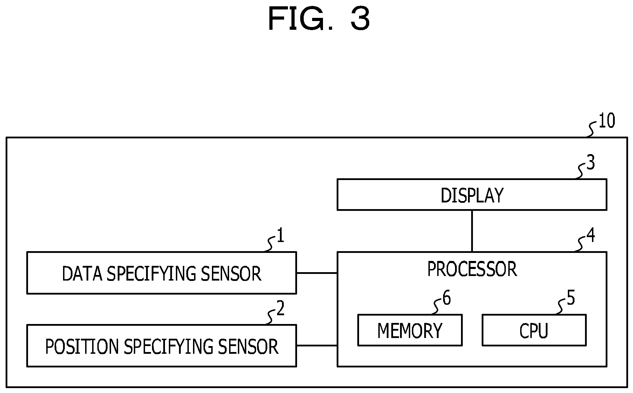

Next, an example of a hardware configuration of an information processing device 10 according to the embodiment of the present invention is described with reference to FIG. 3. The information processing device 10 according to the present embodiment can be applied to a personal computer (PC), a tablet terminal, a smartphone, a personal digital assistants (PDA), and a wearable device.

The information processing device 10 has a processor 4 and a display 3. The processor 4 includes a CPU 5 and a memory 6. The display 3 has a liquid crystal display (LCD) for example as a component, and displays an estimated form and other information.

The CPU 5 is an arithmetic unit that realizes a control of the entire device and an installed function by reading a program and data from the memory 6 and executing a preprocessing, a learning processing, and a form estimation processing.

The memory 6 has a random access memory (RAM), a read only memory (ROM), or a hard disk drive (HDD) for example as a component, and stores various types of tables and various types of programs.

The data specifying sensor 1 is an acceleration sensor for example that is attached to the user and transfers temporally continuous information of an acceleration to the information processing device 10. Data obtained from the data specifying sensor 1 is used for extracting data of the characteristic part where the energy of motion of the action of the user varies as small → large → small.

The position specifying sensor 2 is an acceleration sensor for example that is attached to the user and transfers temporally continuous information of an acceleration to the information processing device 10. Data obtained from the position specifying sensor 2 is used for estimating the form corresponding to the extracted characteristic part.

In the present embodiment, two sensors of the data specifying sensor 1 and the position specifying sensor 2 are used for the convenience of description, however, a single sensor may also be used in the present embodiment. When a single sensor is used, data obtained from the sensor is stored in a data specifying table 23, and used for extracting data of a characteristic part concerning a variation of the action of the user. At the same time, the data obtained from the sensor is stored in a position specifying table 24, and used for estimating the form.

### [System Configuration]

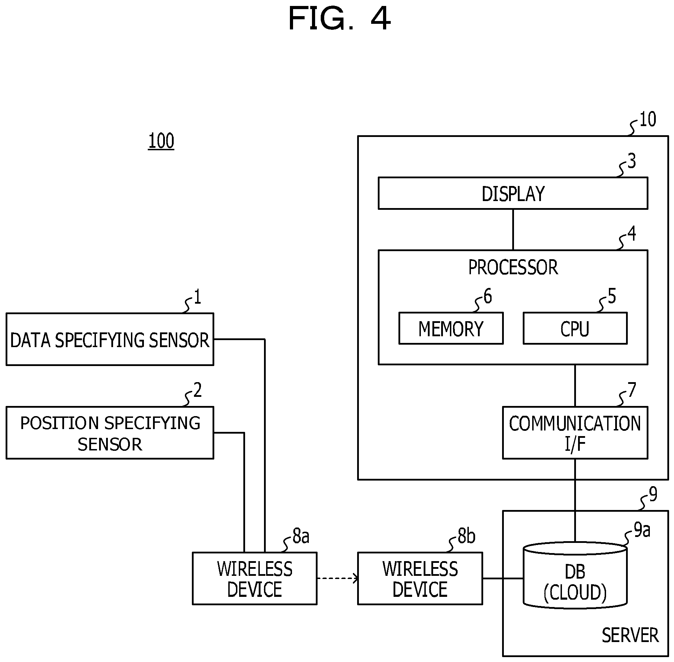

The form estimation to be performed by the present embodiment may be realized by an information processing system 100 illustrated in FIG. 4 as an example. In the information processing system 100 of FIG. 4, the information processing device 10 and a server 9 are connected to each other via a network. In addition, the server 9 is connected to the data specifying sensor 1 and the position specifying sensor 2 via wireless devices 8a and 8b.

The server 9 has a database (DB) 9a that accumulates the information of the acceleration detected by the data specifying sensor 1 and the position specifying sensor 2 The DB 9a may be stored in a storage device inside the server 9, or in a storage device on a cloud. In addition, the server 9 may be an arithmetic unit on a cloud.

In FIG. 4, the information processing device 10 has a communication I/F 7 in addition to the hardware configuration illustrated in FIG. 3. The communication I/F 7 is an interface to connect the information processing device 10 to the network. Consequently, the information processing device 10 can perform data communication with another equipment such as the server 9 via the communication I/F 7. The communication I/F 7 receives necessary data from the DB 9a, and the CPU 5 executes processing for the form estimation using the received data.

### [Functional Configuration of Information Processing Device]

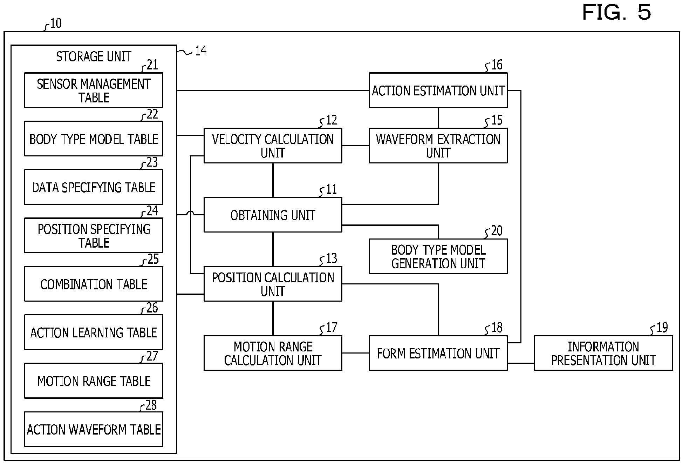

Next, an example of a functional configuration of the information processing device 10 according to the embodiment is described with reference to FIG. 5. The information processing device 10 has an obtaining unit 11, a velocity calculation unit 12, a position calculation unit 13, a storage unit 14, a waveform extraction unit 15, an action estimation unit 16, a motion range calculation unit 17, a form estimation unit 18, an information presentation unit 19, and a body type model generation unit 20.

The obtaining unit 11 obtains temporally continuous sensor data obtained in every 0.1 seconds for example. In the present embodiment, the obtaining unit 11 obtains accelerations detected by the data specifying sensor 1 and the position specifying sensor 2.

The velocity calculation unit 12 integrates the accelerations detected by the data specifying sensor 1 and the position specifying sensor 2, and calculates velocities. The position calculation unit 13 integrates the calculated velocities, and calculate positions.

The storage unit 14 stores a sensor management table 21, a body type model table 22, a data specifying table 23, a position specifying table 24, a combination table 25, an action learning table 26, a motion range table 27, and an action waveform table 28. In addition, the storage unit 14 stores a preprocessing program, a learning processing program, and a form estimation processing program.

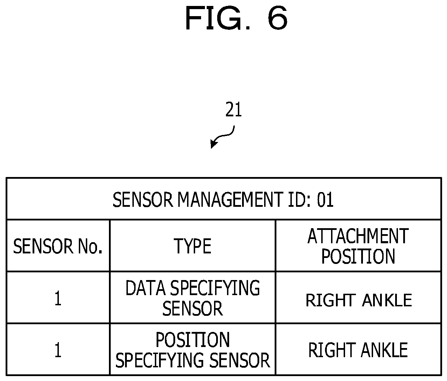

FIG. 6 illustrates an example of the sensor management table 21 according to the embodiment. The sensor management table 21 stores positions of the data specifying sensor 1 and the position specifying sensor 2 that are attached to a user. In the present embodiment, the data specifying sensor 1 and the position specifying sensor 2 are assumed to be worn on the right ankle. In this case, as illustrated in FIG. 6, the sensor management table 21 stores "Right ankle" in the attachment position of the data specifying sensor 1 having a sensor number "1" and in the attachment position of the position specifying sensor 2 having a sensor number "2", in association with a sensor management ID "01".

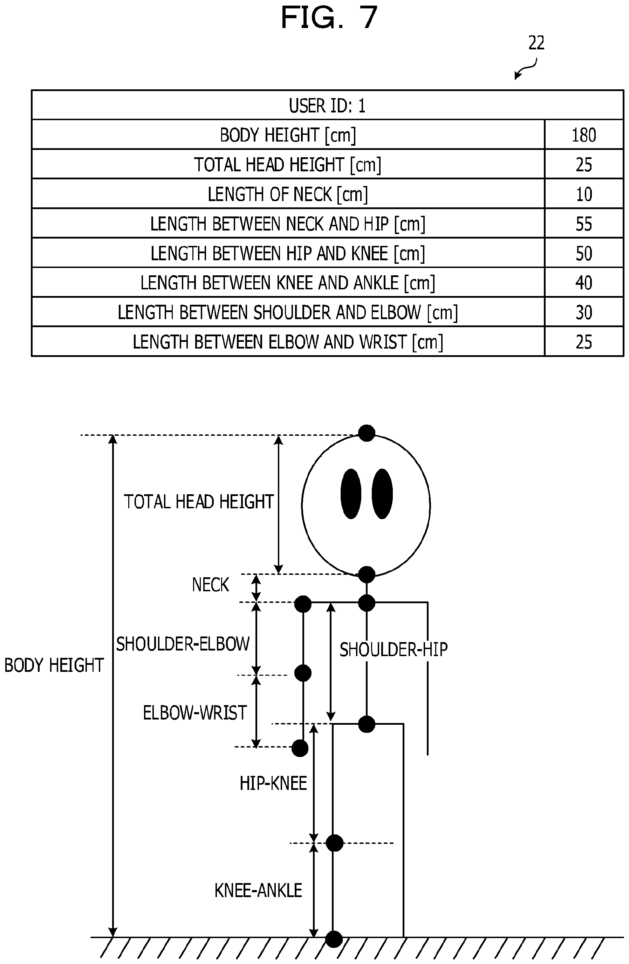

FIG. 7 illustrates an example of a body type model table according to the embodiment. The body type model table 22 stores dimension information concerning the body type of each user. The body type model generation unit 20 obtains dimension information concerning the body type of each user on the basis of an image data or a motion capture, and stores the dimension information in the body type model table 22 beforehand.

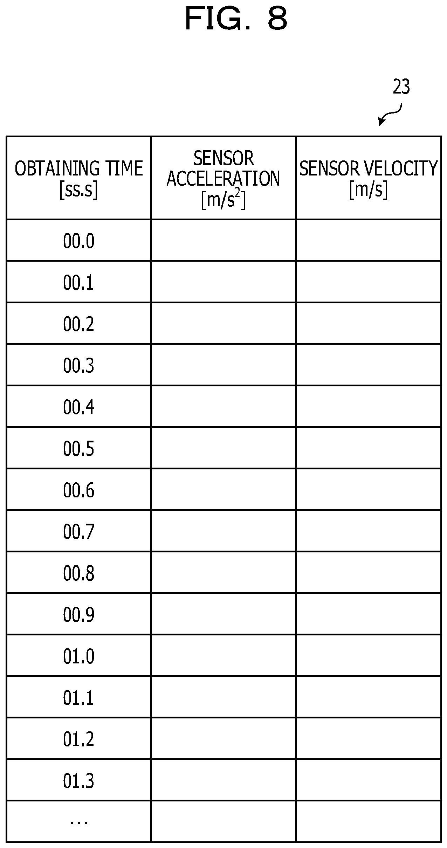

FIG. 8 illustrates an example of the data specifying table 23 according to the embodiment. The data specifying table 23 stores the information of the acceleration detected by the data specifying sensor 1. In addition, the data specifying table 23 stores the information of the velocity obtained by integrating the detected acceleration. The velocity is calculated by the velocity calculation unit 12.

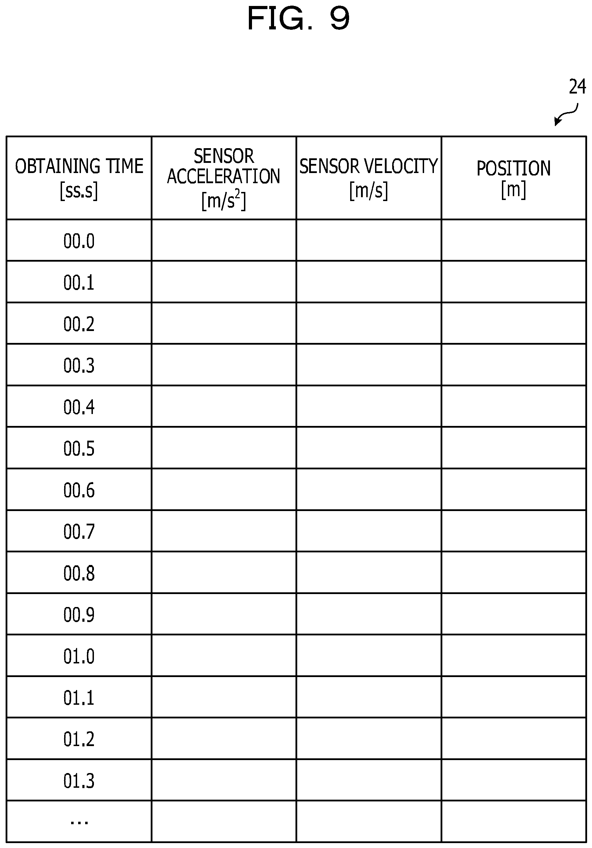

FIG. 9 illustrates an example of the position specifying table 24 according to the embodiment. The position specifying table 24 stores the information of the acceleration detected by the position specifying sensor 2. In addition, the data specifying table 23 stores the information of each of the velocity obtained by integrating the detected acceleration and the position obtained by integrating the velocity. The velocity is calculated by the velocity calculation unit 12. The position is calculated by the position calculation unit 13.

Note that, in the present embodiment, the velocity stored in the data specifying table 23 and the velocity and the position stored in the position specifying table 24 are indicated only with the information of the y axis direction; however, information of the x axis direction and the z axis direction may also be added.

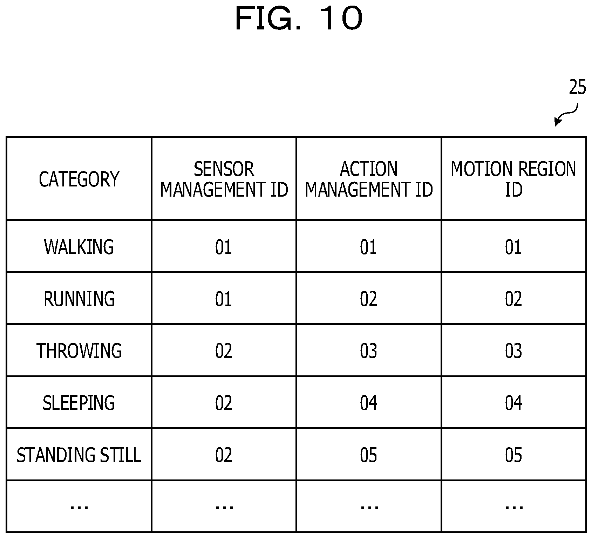

FIG. 10 illustrates an example of the combination table 25 according to the embodiment. The combination table 25 stores a combination of a sensor management ID, an action management ID, and a motion region ID for each category.

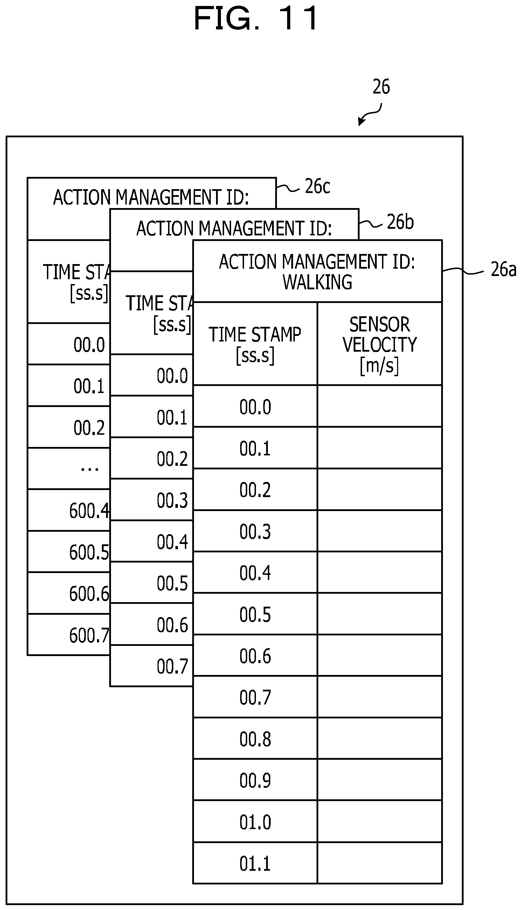

FIG. 11 illustrates an example of the action learning table 26 according to the embodiment. The action learning table 26 stores the information of the velocity calculated from a sensor value obtained in every 0.1 seconds for each category.

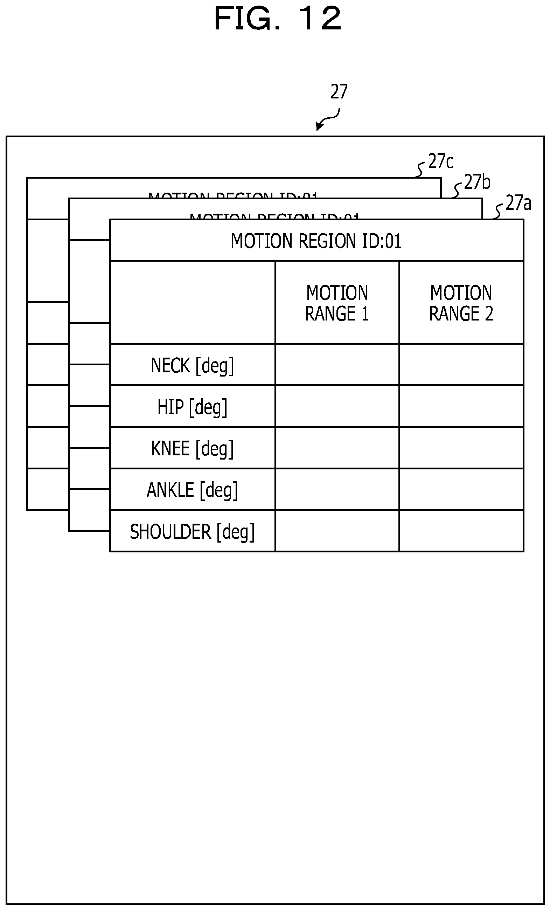

FIG. 12 illustrates an example of the motion range table 27 according to the embodiment. The motion range table 27 stores information of a motion range for each portion of a body. In the present embodiment, the motion range table 27 stores the information of the motion range only for the ankle to which the sensor is attached.

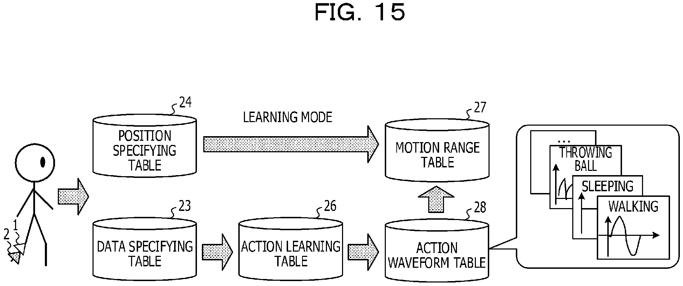

The action waveform table 28 stores actions and waveforms that are learned from sensor values obtained from sensors attached to the user beforehand, in association with each other (refer to FIG. 15). The waveform may also be stored with time series values.

Returning to FIG. 5, the waveform extraction unit 15 narrows down a data area for use on the basis of the acceleration detected by the data specifying sensor 1, and extracts a waveform of the data of the narrowed down range. Narrowing down of the data is performed by extracting a part where the characteristic part of the variation of the energy of motion transitions as small → large → small, that is a part where the velocity relatively transitions from a low velocity to a high velocity and then to a low velocity, from the velocity obtained by integrating the acceleration.

The action estimation unit 16 estimates the action of the user using the data of the range narrowed down with respect to the obtained sensor data.

The motion range calculation unit 17 calculates a joint motion range for performing the estimated action depending on a body type model of the user. The form estimation unit 18 estimates the form of the user on the basis of the calculated joint motion range and the calculated position information.

The information presentation unit 19 indicates the estimated form of the user. The body type model generation unit 20 generates a body type model of the user on the basis of the body type information of the user.

Functions of the obtaining unit 11, the velocity calculation unit 12, the position calculation unit 13, the waveform extraction unit 15, the action estimation unit 16, the motion range calculation unit 17, the form estimation unit 18, the information presentation unit 19, and the body type model generation unit 20 are realized through processing of the preprocessing program, the learning processing program, and the form estimation processing program stored in the memory 6 for example to be executed by the CPU 5. Function of the storage unit 14 is realized by the memory 6 for example. Function of the information presentation unit 19 is realized by the display 3 for example.

### [Preprocessing]

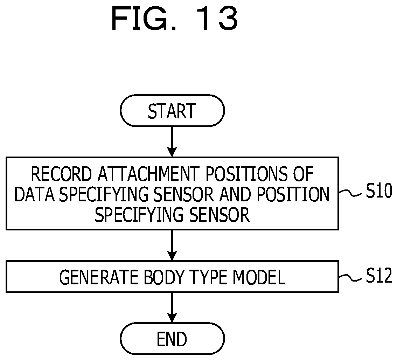

Next, an example of the preprocessing according to the present embodiment is described with reference to FIG. 13. FIG. 13 is a flowchart that illustrates an example of the preprocessing according to the embodiment. When the processing is started, the obtaining unit 11 obtains the attachment positions of the data specifying sensor 1 and the position specifying sensor 2 that are attached to the user, and stores the attachment positions in the sensor management table 21 (step S10). In the present embodiment, the data specifying sensor 1 and the position specifying sensor 2 are attached to the right ankle of the user.

Next, the body type model generation unit 20 generates dimension information concerning the body type of the user, stores the dimension information in the body type model table 22 (step S12), and ends the processing.

### [Learning Processing]

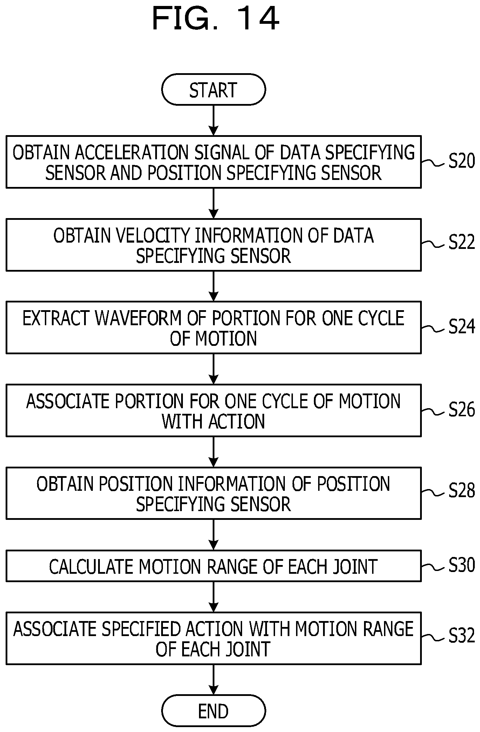

Next, an example of the learning processing according to the present embodiment is described with reference to FIG. 14. FIG. 14 is a flowchart that illustrates an example of the learning processing according to the embodiment. In the learning processing, by sensing daily actions of the user, waveforms of individual actions of the user (velocity data group) are learned as illustrated in FIG. 15 as an example.

When the processing is started, the obtaining unit 11 obtains signals of the accelerations from the data specifying sensor 1 and the position specifying sensor 2 (step S20). The acceleration obtained by the data specifying sensor 1 is stored in the data specifying table 23, and the acceleration of the position specifying sensor 2 obtained by the position specifying table 24 is stored in the position specifying table 24.

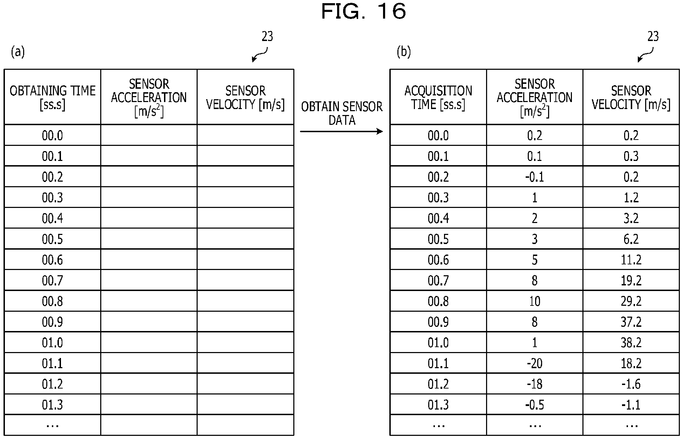

Next, the velocity calculation unit 12 calculates a velocity from the acceleration obtained from the data specifying sensor 1, and stores the calculated velocity in the data specifying table 23 (step S22). FIG. 16 illustrates an example in which, in the initialized data specifying table 23 of FIG. 16(a), information of the acceleration obtained from the data specifying sensor 1 and the calculated velocity are stored as illustrated in FIG. 16(b).

Returning to FIG. 14, the waveform extraction unit 15 extracts a waveform of a portion for one cycle of motion (step S24). In other word, the waveform extraction unit 15 extracts information of the characteristic part of the velocity that varies as low → high → low from the information of the velocity of the data specifying table 23. Because it is a learning phase, the waveform of the portion for one cycle of motion may be extracted manually, or the waveform may be extracted from a frequency spectrum.

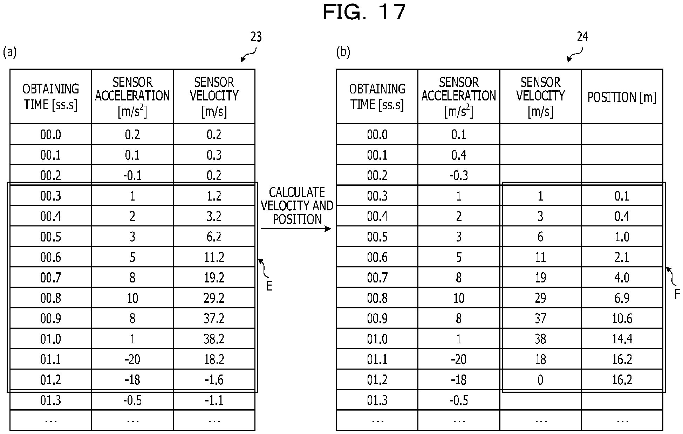

As a result, information E of the portion for one cycle of motion where the velocity relatively transitions from a low velocity to a peak velocity and then a low velocity is extracted as illustrated in FIG. 17(a). The information E of the portion for one cycle corresponds to a data group of the velocity that varies as low → high → low illustrated in the upper portion of the range A in FIG. 2 for example.

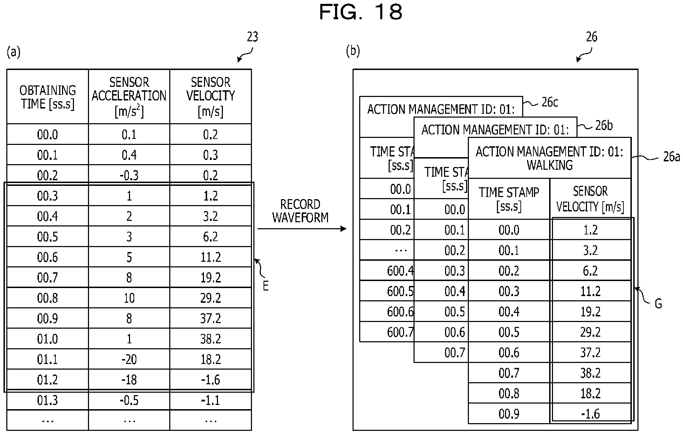

Returning to FIG. 14, the action estimation unit 16 associates the information of the portion for one cycle of motion with an action (walking, throwing a ball, or the like), and registers the information and the action in the action learning table 26 (step S26). For example, the action estimation unit 16 associates the information E of the portion for one cycle of motion in the data specifying table 23 illustrated in FIG. 18(a) having the same content as that in FIG. 17(a), with a "walking" action being performed by the user, and stores the information E and the action in the action learning table 26 as illustrated in FIG. 18(b). Furthermore, the waveform of the information E of the portion for one cycle associated with the action may be specified and stored in the action waveform table 28 as a waveform associated with the action. FIG. 15 illustrates an example in which the information of the portion for one cycle of motion in the data specifying table 23 is stored in the action learning table 26, and the waveform indicated by the information of the portion for one cycle of motion is stored in the action waveform table 28.

Returning to FIG. 14, the position calculation unit 13 calculates a velocity and a position by integration using information of an acceleration corresponding to the information E of the portion for one cycle of motion extracted from the data specifying table 23 among the information of the acceleration stored in the position specifying table 24 (step S28). The storage unit 14 stores the information of each of the calculated velocity and position in the position specifying table 24 as illustrated in FIG. 17(b).

The information stored in the data specifying table 23 is used for extracting a waveform of an action. Therefore, when a velocity is calculated from an acceleration in the data specifying table 23, the velocity is calculated on the basis of all of the acceleration in the data specifying table 23.

On the other hand, information stored in the position specifying table 24 is used for obtaining a position of a sensor attached to the user. Therefore, when a velocity is calculated from an acceleration in the position specifying table 24, the velocity is calculated by integrating only the acceleration included in information F of FIG. 17(b) that corresponds to the information E of FIG. 17(a). In addition, a position is calculated by integrating the calculated velocity. As described above, in the calculation of a position, a possibility where a noise is included in the calculated position can be reduced further by not using accelerations other than an acceleration that is included in the extracted information F.

Returning to FIG. 14, next, the motion range calculation unit 17 obtains information of the position obtained from the information of the portion for one cycle of motion extracted from the position specifying table 24, calculates a motion range of a joint on the basis of the obtained information of the position, and stores the motion range in the motion range table 27 (step S30).

For example, in a case of the data specifying table 23 of FIG. 17(a) and the position specifying table 24 of FIG. 17(b), the information F of the portion for one cycle of motion in the position specifying table 24 that corresponds to the information E of the portion for one cycle of motion extracted from the data specifying table 23 has the same obtaining times of accelerations detected by the data specifying sensor 1 and the position specifying sensor 2 as those in the information E extracted from the data specifying table 23. However, the information of the portion for one cycle of motion in the position specifying table 24 that corresponds to the information of the portion for one cycle of motion extracted from the data specifying table 23 is not always the case above. For example, the information of the portion for one cycle of motion in the position specifying table 24 that corresponds to the information of the portion for one cycle of motion extracted from the data specifying table 23 may have either the same or almost the same obtaining times of accelerations detected by the data specifying sensor 1 and the position specifying sensor 2 as those in the information extracted from the data specifying table 23.

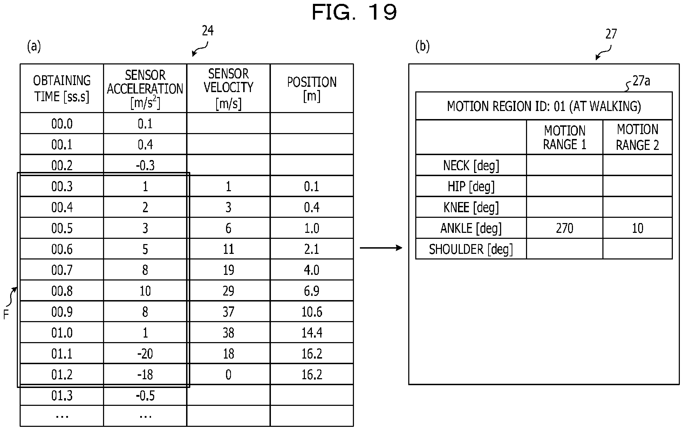

In the motion range table 27, an initial entry of 0° to 360° is set for a motion range of an ankle. In the case of the present embodiment, when step S30 is executed, a motion range of 10° to 270° for the ankle attached with the sensor corresponding to the position information of the information F of the portion for one cycle of motion in the position specifying table 24 of FIG. 19(a), for example, is stored in the column of the ankle of the motion range table 27. Note that when the sensor is attached to a portion other than the ankle, a motion range is individually specified for the portion attached with the sensor, and the motion range is stored in the column of the corresponding portion in the motion range table 27.

Note that step S30 may be omitted. In this case, the motion range of the portion attached with the sensor is indicated with position information of the information F of the portion for one cycle of motion in the position specifying table 24.

Returning to FIG. 14, the motion range calculation unit 17 associates the specified action associated with one cycle of motion in step S26, with the calculated motion range of each joint, stores the specified action and the calculated motion range in the combination table 25 (step S32), and ends the processing. Consequently, as illustrated in the first row in the combination table 25 of FIG. 10 for example, a combination of a category of "walking", a sensor management ID of "01", an action management ID of "01", and a joint motion region ID of "01" is stored.

### [Form Estimation Processing]

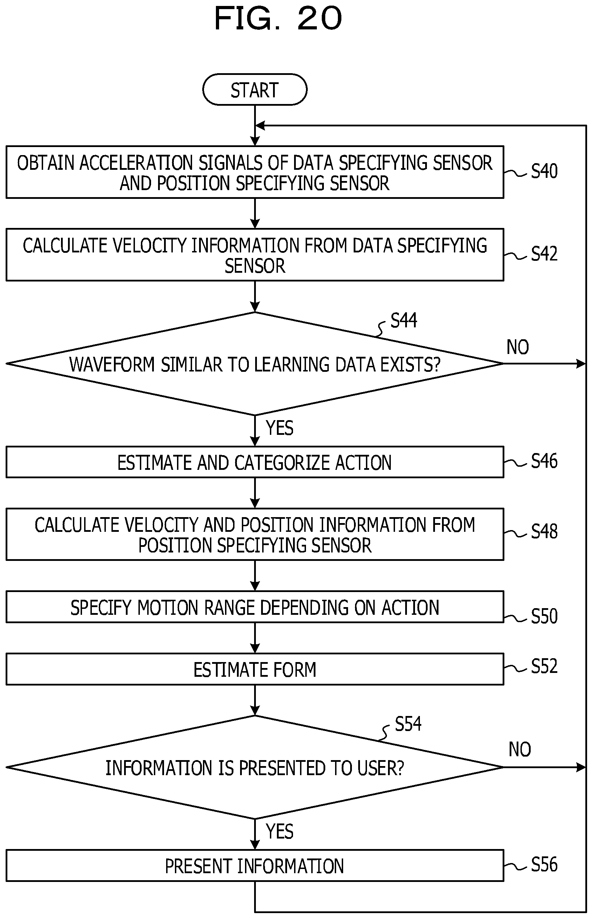

Next, an example of the form estimation processing according to the present embodiment is described with reference to FIG. 20. FIG. 20 is a flowchart that illustrates an example of the form estimation processing according to the embodiment. The form estimation processing estimates an action of the user using the sensor attached to the user, and estimates a form on the basis of the estimated action. In the present embodiment, the already-obtained information of the body type model of the user, and the data specifying sensor 1 and the position specifying sensor 2, are used in this processing. Note that a single sensor may also be used in this processing. In that case, an acceleration detected by the single sensor is stored in the data specifying table 23 and the position specifying table 24.

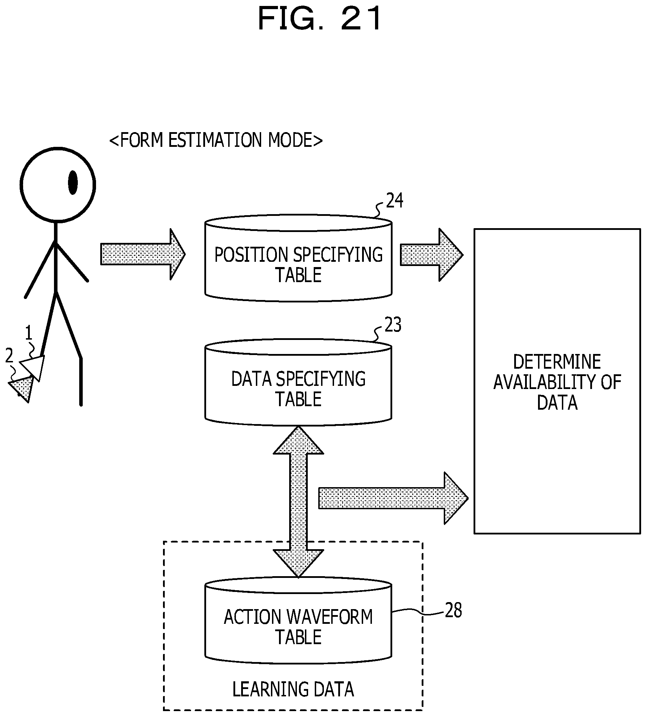

The form estimation processing is real-time processing, and as illustrated in FIG. 21, in the present embodiment, the user attaches two sensors (the data specifying sensor 1 and the position specifying sensor 2) on the right ankle to perform sensing with the both sensors. Information of an acceleration detected by the data specifying sensor 1 is stored in the data specifying table 23, and information of an acceleration detected by the position specifying sensor 2 is stored in the position specifying table 24.

Furthermore, in each time of the action of the user, the form estimation processing includes performing a comparison between a waveform of a short-term form in the action waveform table 28 (or the action learning table 26) learned beforehand and the waveform of the short-term form stored in the data specifying table 23 this time. Consequently, information to be able to be used in the position specifying table 24 is limited for each waveform delimited by the short-term form, and the form is estimated on the basis of the limited information. Accordingly, by removing information to become the noise in the estimation of the form from the information stored in the position specifying table 24, a correct form can be estimated.

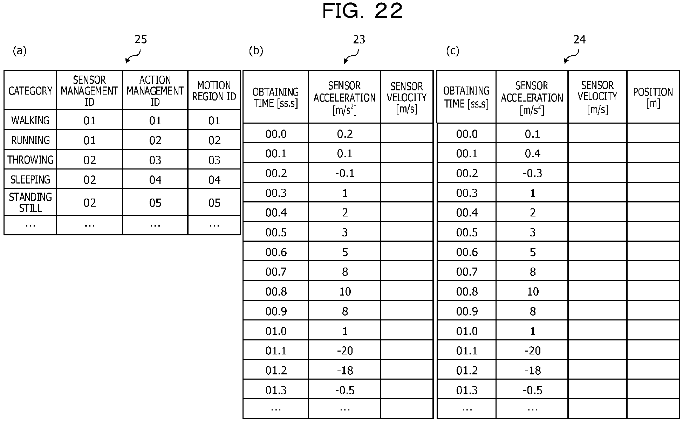

Before starting the processing, the obtaining unit 11 reads the combination table 25 illustrated in FIG. 22(a), and obtains each of a sensor management ID, action management ID, and motion region ID of a corresponding category. In addition, the data specifying table 23 and the position specifying table 24 are initialized.

When the processing is started, the obtaining unit 11 obtains signals of accelerations from the data specifying sensor 1 and the position specifying sensor 2 (step S40). As illustrated in FIG. 22(b), the acceleration obtained by the data specifying sensor 1 is stored in the data specifying table 23. In addition, as illustrated in FIG. 22(c), the acceleration of the position specifying sensor 2 obtained by the position specifying table 24 is stored in the position specifying table 24. Next, the velocity calculation unit 12 calculates a velocity from the acceleration obtained from the data specifying sensor 1, and stores the calculated velocity in the data specifying table 23 (step S42).

Next, the action estimation unit 16 performs a comparison between the information of the velocity stored in the data specifying table 23 and the information of the velocity for each category stored in the action learning table 26. As a result of the comparison, the action estimation unit 16 determines whether a waveform similar to the information of the velocity (learning data) stored in the action learning table 26 exists in the information of the velocity stored in the data specifying table 23 (step S44).

The action estimation unit 16, when determining that a waveform similar to the information of the velocity stored in the action learning table 26 exists in the information of the velocity stored in the data specifying table 23, estimates that the category indicated by the information of the velocity is the action of the user (step S46).

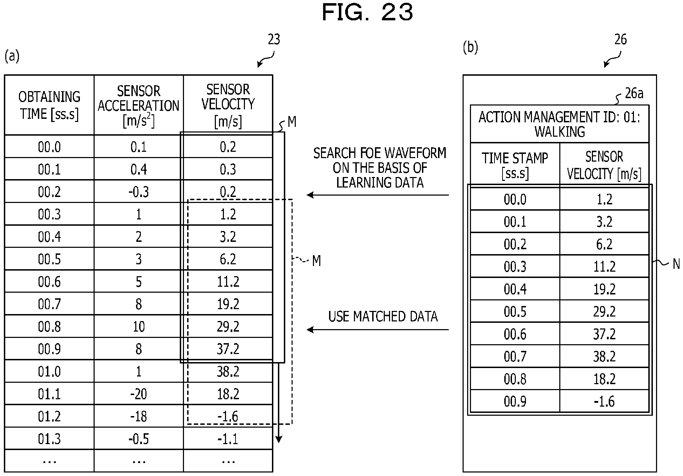

For example, by sequentially matching information N of the velocities stored in the action learning table 26 illustrated in FIG. 23(b) to the velocities stored in the data specifying table 23 illustrated in FIG. 23(a), information M of the velocities enclosed by a dotted line matches the information N. In this case, a waveform similar to the information of the velocities stored in the action learning table 26 is determined to exist in the information of the velocities stored in the data specifying table 23, and "Walking", which is a category of the action corresponding to the information N of the velocities stored in the action learning table 26, is estimated to be the action of the user.

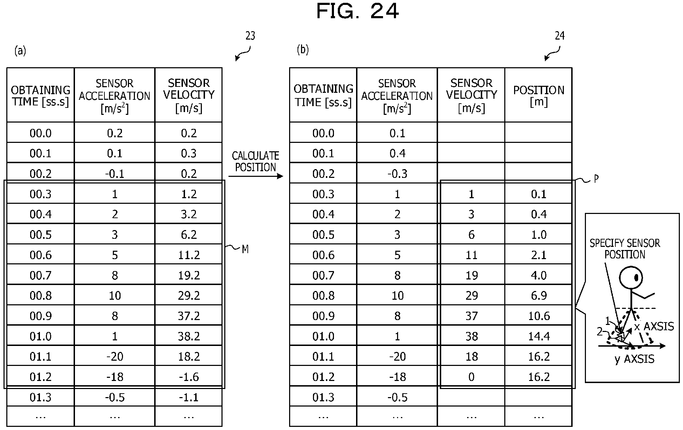

Returning to FIG. 20, next, the velocity calculation unit 12 calculates a velocity by integrating the acceleration stored in the position specifying table 24 and then calculates a position by integrating the velocity, and stores the information of the velocity and position in the position specifying table 24 (step S48). At this time, the velocity calculation unit 12 does not include accelerations other than the accelerations included in the information M extracted from the data specifying table 23 of FIG. 24(a), in the integration target, and integrates only accelerations included in information P in the position specifying table 24 of FIG. 24(b) corresponding to the information M. The position is calculated by integrating the velocity calculated above. Consequently, the noise can be reduced in the acceleration detected by the sensor.

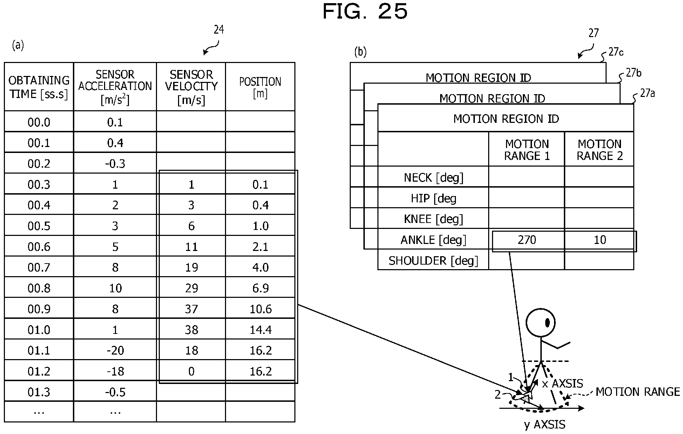

Next, the motion range calculation unit 17 specifies a motion range depending on the estimated action based on the motion range table 27 (step S50). In the case of the motion range table 27 illustrated in FIG. 25(b), the motion range calculation unit 17 specifies a motion range of 10° to 270° of the ankle of the user with the sensor attached depending on the estimated action of "walking".

Note that step S50 may be omitted. In this case, the form may be determined based on position information included in the information P of the portion for one cycle of motion in the position specifying table 24.

Returning to FIG. 20, next, the form estimation unit 18 estimates a form concerning a walking action carried out within the specified motion range of 10° to 270° of the ankle on the basis of information of the position included in the information P extracted from the position specifying table 24 (step S52). Next, the information presentation unit 19 determines whether the estimated form is to be presented to the user (step S54).

The information presentation unit 19, when determining not to present the estimated form to the user, immediately returns to step S40, and repeats the processing from step S40. Conversely, the information presentation unit 19, when determining to present the estimated form to the user, displays the estimated form in a terminal or the like of the user (step S56), returns to step S40, and repeats the processing from step S40.

As described above, with the information processing device 10 according to the embodiment, temporally continuous sensor data can be obtained and the form of a living object (a temporally continuous posture) can be estimated. In particular, the variation of the motion that transitions as small → large → small is taken as an amount of characteristic, and data to be used and data not to be used for estimating the action and the form are distinguished among the sensor data. Consequently, accuracy in estimating the form can be improved. In other word, by extracting the characteristic part where the walking velocity of the user transitions as low → high → low from the sensor data detected by the sensor attached to the user, the range of a short-term energy variation specific to the motion is specified, and the action of the user is quantified by using only the sensor data within the specified range. Consequently, the action of the user can be quantified accurately in the state where the noise is removed from the sensor data. As a result, the form of the user can be estimated correctly.

Therefore, by using the information processing device 10 according to the present embodiment, an effect of rehabilitation can be measured not only in the hospital or a rehabilitation institution, but also at home. In other words, by attaching the wearable sensors to the user and measuring daily actions of the user, the actions of the user attached with the sensors can be quantified, and the forms of the actions can be estimated accurately. In addition, separately attaching the wearable sensors to a plurality of joints of the user enables visualization of the form of the entire body. In particular, the visualization can support a case that a user who is hospitalized due to a bone fracture or another reason to recover, through the rehabilitation, the optimal form (a continuous posture) before getting injured.

As a method for displaying the form estimated in step S56 of FIG. 20 for the user to easily understand the optimal form, the correct form may also be displayed by superimposing the correct form on the estimated form. Consequently, the user can see the shape of its own form more precisely, and understand points to be improved on its own form more easily.

In addition, a form of the user before getting injured may also be displayed by superimposing the form of the user before getting injured on a form of the user after getting injured. Consequently, a supporter can give appropriate advices to the user for recovering the original form while pointing out differences of the forms before and after getting injured on a screen. In addition, the user itself can see its own form objectively, and find points to be improved on the form more easily. In the case that the user is a rehabilitant, the user itself can know the degree of its own recovery by seeing a display of the forms before and after getting injured, and be encouraged for future rehabilitation.

Furthermore, by connecting the information processing device 10 according to the present embodiment to a server and a terminal in a hospital via a network, information of the estimated form can be provided to a person concerned in the hospital. Consequently, the person concerned in the hospital can see the current state of the user, and use the information for making policies on the future rehabilitation and treatment of the user.

Although the information processing device, the information processing system, and the information processing method have been described with reference to the above embodiments, the information processing device, the information processing system, and the information processing method according to the present invention are not limited to the above-described embodiment, and various modifications and improvements can be made within the scope of the present invention. In addition, when there are multiple embodiments and modifications described above, they can be combined within a range that does not contradict each other.

For instance, a sensor to be used in the present invention is not limited to a wearable sensor such as an acceleration sensor, but a camera or an infrared ray sensor capable of shooting a specific portion of the user may also be used. The present embodiment may also be applied by extracting an outline, a characteristic part, or the like of the subject from image data obtained by using the camera or the infrared ray sensor, and calculating a velocity that is a differential value of a position on the basis of the outline or a movement of the position of the characteristic part. In addition, although a human is taken as the example of the subject for the form estimation in the embodiment above, the subject is not limited to a human, but may be an animal such as a pet.

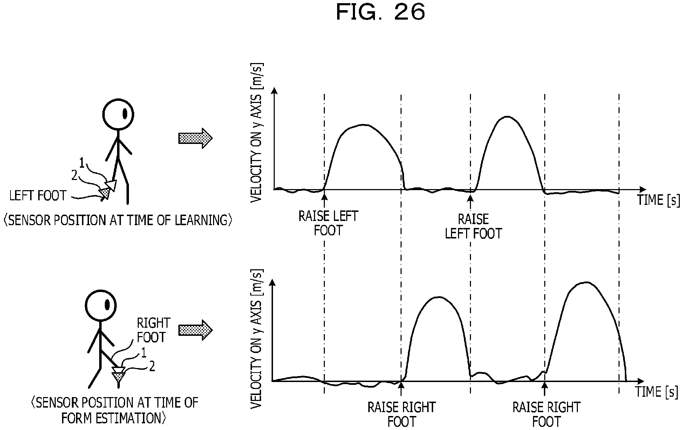

In the embodiment above, the user attached the sensors on the same portion of the body both at the time of learning and at the time of form estimation. However, a case where the user attaches the sensors on different portions between the time of learning and the time of form estimation is presumed. An example of the case is when the user attaches the data specifying sensor 1 and the position specifying sensor 2 on the left ankle at the time of learning as illustrated in the upper part of FIG. 26, and on the right ankle at the time of form estimation as illustrated in the lower part of FIG. 26.

In this case, appearing time of a characteristic part having a variation as small → high → small in a velocity calculated from accelerations obtained from the data specifying sensor 1 and the position specifying sensor 2 deviates by a half step between the velocity on the y axis at the time of learning in the upper portion of FIG. 26 and the velocity on the y axis at the time of form estimation in the lower part of FIG. 26.

Therefore, in this case, information of the portion for one cycle of motion in the position specifying table 24 corresponding to information of the portion for one cycle of motion extracted from the data specifying table 23 does not have the same obtaining times of accelerations detected by the data specifying sensor 1 and the position specifying sensor 2 as those in the information extracted from the data specifying table 23. In this case, the information of the portion for one cycle of motion in the position specifying table 24 deviates by a half step from the information of the portion for one cycle of motion for the characteristic part extracted from the data specifying table 23. In the case of FIG. 26, with respect to information Q of the portion for one cycle of motion extracted from the data specifying table 23, information R of the portion for one cycle of motion in the position specifying table 24 deviating by a half step is extracted.

In another case where sensors are attached to different portions such as the wrist and the ankle between the time of learning and the time of form estimation, information of the portion for one cycle of motion in the position specifying table 24 corresponding to information of the portion for one cycle of motion extracted from the data specifying table 23 is extracted taking into account a deviation in the time or a deviation in the amplitude of a velocity having been determined beforehand.

### REFERENCE SIGNS LIST

- 1: Data specifying sensor

- 2: Position specifying sensor
- 3: Display
- 4: Processor
- 5: CPU
- 6: Memory
- 7: Communication I/F
- 9: Server
- 10: Information processing device
- 11: Obtaining unit
- 12: Velocity calculation unit
- 13: Position calculation unit
- 14: Storage unit
- 15: Waveform extraction unit
- 16: Action estimation unit
- 17: Motion range calculation unit
- 18: Form estimation unit
- 19: Information presentation unit
- 20: Body type model generation unit
- 21: Sensor management table
- 22: Body type model table
- 23: Data specifying table
- 24: Position specifying table
- 25: Combination table
- 26: Action learning table
- 27: Motion range table
- 28: Action waveform table

## Claims

1. An information processing device comprising:
an extraction unit that extracts data of a characteristic part concerning a variation of a movement of a subject from data that is obtained from a sensor attached to the subject and indicates the movement of the subject;
an action estimation unit that estimates an action of the subject with reference to a storage unit that stores an action pattern of the subject, the action corresponding to the extracted data of the characteristic part; and
a form estimation unit that estimates a form of the estimated action of the subject, the form corresponding to the extracted data of the characteristic part.

2. The information processing device according to claim 1, wherein
the extraction unit extracts data of a characteristic part where a velocity of a movement of the subject relatively transitions from a low velocity to a high velocity and then again to a low velocity.

3. The information processing device according to claim 1, wherein
the extraction unit extracts data of a characteristic part concerning a variation of a movement of the subject beforehand from data that is different from the obtained data and indicates a movement of the subject, the data being obtained from a sensor attached to the subject; and
the storage unit stores an action pattern of the subject, the action pattern being obtained by associating the data of the characteristic part extracted beforehand with the action of the subject at a time of obtaining the data.

4. An information processing system in which a server and an information processing device are connected to each other, the information processing device comprising:
an extraction unit that extracts data of a characteristic part concerning a variation of a movement of a subject from data that is obtained from a sensor attached to the subject and indicates the movement of the subject;
an action estimation unit that estimates an action of the subject with reference to a storage unit that stores an action pattern of the subject, the action corresponding to the extracted data of the characteristic part; and
a form estimation unit that estimates a form of the estimated action of the subject, the form corresponding to the extracted data of the characteristic part.

5. The information processing system according to claim 4, wherein
the extraction unit extracts data of a characteristic part where a velocity of a movement of the subject relatively transitions from a low velocity to a high velocity and then again to a low velocity.

6. The information processing system according to claim 4, wherein
the extraction unit extracts data of a characteristic part concerning a variation of a movement of the subject beforehand from data that is different from the obtained data and indicates the movement of the subject, the data being obtained from the sensor attached to the subject; and
the storage unit stores an action pattern of the subject, the action pattern being obtained by associating the data of the characteristic part extracted beforehand with the action of the subject at a time of obtaining the data.

7. An information processing method wherein a computer executes:
extraction of data of a characteristic part concerning a variation of a movement of a subject from data indicating a movement of the subject obtained from a sensor attached to the subject;
estimation of an action of the subject with reference to a storage unit that stores an action pattern of the subject, the action corresponding to the extracted data of the characteristic part; and
estimation of a form of the estimated action of the subject, the form corresponding to the extracted data of the characteristic part.

8. The information processing method according to claim 7, comprising
extracting data of a characteristic part where a velocity of a movement of the subject relatively transitions from a low velocity to a high velocity and then to a low velocity.

9. The information processing method according to claim 7, comprising
extracting data of a characteristic part concerning a variation of a movement of the subject beforehand from data that is different from the obtained data and indicates a movement of the subject, the data being obtained from a sensor attached to the subject; and
storing an action pattern of the subject in the storage unit, the action pattern being obtained by associating the data of the characteristic part extracted beforehand with the action of the subject at a time of obtaining the data.
